# FASCICULE DE BREVET EUROPEEN

(11) **EP 4 168 386 B1**
(45) Date de publication et mention de la délivrance du brevet: **18.02.2026**
(21) Numéro de dépôt: 21740119.9
(22) Date de dépôt: 16.06.2021
(51) Int. Cl.: C07C 227/08, C07C 229/08

(54) **PROCEDE DE FABRICATION D'ACIDE AMINOUNDECANOIQUE ET D'ACIDE AMINODECANOIQUE**
VERFAHREN ZUR HERSTELLUNG VON AMINOUNDECANSÄURE UND AMINODECANSÄURE
METHOD FOR PRODUCING AMINOUNDECANOIC ACID AND AMINODECANOIC ACID

(30) Priorité: 18.06.2020 FR 2006390
(43) Date de publication de la demande: 26.04.2023
(73) Titulaire: ARKEMA FRANCE, 92800 Puteaux (FR)
(72) Inventeur: DEVAUX, Jean-François, 69493 PIERRE-BENITE CEDEX (FR); GERBIER, Myriam, 69493 PIERRE-BENITE CEDEX (FR); BOSSOUTROT, Jean-Michel, 69493 PIERRE-BENITE CEDEX (FR); OROZCO, Jose Luis, 69492 PIERRE-BENITE CEDEX (FR)
(74) Mandataire: Bandpay & Greuter
(86) Numéro de dépôt international: PCT/FR2021/051080
(87) Numéro de publication internationale: WO 2021/255387

(56) Documents cités:
- WO-A1-2018/080869

## Description

### [Domaine technique]

La présente demande de brevet concerne un procédé de fabrication d'acides aminocarboxyliques, notamment d'acide 10-aminodécanoïque, d'acide 11-aminoundécanoïque et d'acide 12-aminododécanoïque.

### [Technique antérieure]

Les acides aminocarboxyliques comme l'acide 10-décanoïque, l'acide 11-aminoundécanoïque et d'acide 12-aminododécanoïque sont des monomères utiles notamment dans la fabrication de polyamides à longues chaînes comme le polyamide 10, le polyamide 11 et le polyamide 12, respectivement.

Les excellentes propriétés thermiques et mécaniques de ces polyamides ainsi que leur résistance chimique les rendent intéressants pour diverses applications, notamment dans l'automobile, les équipements sportifs, les revêtements ou l'impression 3D. Un avantage de plus en plus apprécié de ces polyamides est le fait que leurs monomères peuvent être obtenus à partir de produits d'origine végétale et donc renouvelables.

Ainsi, l'acide 11-aminoundécanoïque est accessible à partir d'huile de ricin par transestérification en présence de méthanol et pyrolyse subséquente. Le 10-undécénoate de méthyle obtenu est hydrolysé en acide 10-undécénoïque qui peut être bromuré en bout de chaîne par une étape d'hydrobromuration. L'acide 11-bromoundécanoïque (ou ω-bromoundécanoïque) obtenu peut alors être soumis à une étape d'ammonolyse par réaction avec l'ammoniac pour donner l'acide 11-aminoundécanoïque et du bromure d'ammonium.

L'étape d'hydrobromuration est décrite par exemple dans le brevet français FR 928265 A. On fait agir de l'acide bromhydrique sur l'acide 10-undécénoïque en solution dans un solvant organique en présence d'un catalyseur tel que le peroxyde de benzoyle.

Le brevet français FR 951932 propose d'augmenter le rendement en acide 11-bromoundécanoïque à 80% environ en utilisant l'acide bromhydrique en excès et un solvant hydrocarbure tel que le benzène, le toluène, l'éther de pétrole ou des hydrocarbures chlorés.

Selon le brevet européen EP 3 030 543 B1, on peut utiliser également d'autres solvants lorsque la réaction est conduite en deux étapes, la première étape consistant à mélanger dans un premier réacteur l'acide 10-undécénoïque et l'acide bromhydrique sous forme de flux liquide en régime turbulent et la seconde étape consistant à poursuivre la réaction dans un second réacteur en régime transitoire ou laminaire.

Les demandes de brevet FR 928265 et EP 235 866 B1 décrivent la conversion de l'acide 11-bromoundécanoïque en acide 11-aminoundécanoïque par un traitement avec une solution aqueuse d'ammoniaque. L'acide 11-aminoundécanoïque formé est séparé du bromure d'ammonium formé. La demande WO 2018/080869 A1 décrit la synthèse d'acide 10-aminodécanoïque à partir d'acide 9-décénoïque obtenu à partir d'une composition d'huile de palme. En particulier l'hydrobromuration d'acide 9-décénoïque est réalisée par bullage de bromure d'hydrogène gazeux à travers une solution d'acide 9-décénoique dans le toluène en présence de peroxyde de benzoyle à une température de 5 à 15°C. Après lavage, séchage et purification dans l'hexane, le rendement d'acide 10-bromodécanoïque est de 64%. L'acide 10-bromodécanoïque peut réagir avec l'ammoniaque à 28% à température ambiante puis à 45°C pour former l'acide 10-aminodécanoïque, qui est récupéré par cristallisation à température ambiante puis filtration lavage et séchage.

Il est connu de l'homme de l'art que l'acide 11-dodécénoïque, fabriqué à partir d'acide 10-undécénoïque peut réagir avec le bromure d'hydrogène en présence d'hydroperoxyde de benzoyle pour donner l'acide 12-bromododécanoïque avec un rendement de 88% (voir Chemistry & Industry (London) 1954, 190-191).

Le document WO 2018/0808 décrit la préparation d'acide 10-aminodécanoïque par réaction d'acide décénoïque avec du bromure d'hydrogène suivi d'une réaction avec l'ammoniaque.

L'ensemble de ces procédés présentent comme inconvénient de requérir des quantités substantielles de bromure d'hydrogène, généralement produit sur site par réaction de brome onéreux avec de l'hydrogène, et de produire des quantités importantes d'effluents chargés en sels.

Il est connu de transformer le bromure d'ammonium obtenu à l'étape d'ammonolyse en bromure d'hydrogène, via une conversion en bromure de sodium puis en brome, lequel est réagi ensuite avec l'hydrogène (voir Kunststoff Handbuch, Polyamide, 3/4, édité par L. Bottenbruch et R. Binsack, Hanser Fachbuchverlag 1998, pp. 646-648).

Malgré les progrès, il reste cependant encore le besoin d'améliorer ce procédé. En particulier, on observe des rendements peu élevés pour la conversion de bromure de sodium en brome et des encrassements lors de la conversion du brome ainsi obtenu en bromure d'hydrogène.

### [Résumé de l'invention]

L'invention a donc pour but de proposer un procédé de fabrication d'acides aminocarboxyliques, notamment d'acide 12-aminododécanoïque, d'acide 11-aminoundécanoïque et d'acide 10-aminodécanoïque plus économique et permettant de réduire la quantité d'effluents.

En effet, la présente invention repose sur la constatation qu'il est possible de réduire la quantité de brome requis pour le procédé ainsi que celle des bromures rejetées dans l'environnement en remplaçant une partie du brome par du chlore.

Par ailleurs, il a été découvert qu'une étape de purification du bromure de sodium avant la conversion en brome permettait de réduire substantiellement l'encrassement du réacteur lors de la transformation en bromure d'hydrogène.

Aussi, selon un premier aspect, l'invention a pour objet un procédé de production d'acide aminocarboxylique de formule (I) suivante :

NH₂-CH₂-(CH₂)ₙ-COOH (I)

dans laquelle n est un entier de 7 à 12, de préférence de 8 à 10, comprenant les étapes suivantes :
(i) réaction de l'acide carboxylique insaturé de formule (II) suivante :

   CH₂=CH-(CH₂)ₙ₋₁-COOH (II)

   avec du bromure d'hydrogène (HBr) pour former l'acide ω-bromoalcanoïque de formule (III) suivante :

   Br-CH₂-(CH₂)ₙ-COOH (III) ;
(ii) réaction de l'acide ω-bromoalcanoïque de formule (III) obtenu avec l'ammoniac en solution aqueuse pour former un mélange réactionnel comprenant l'acide aminocarboxylique de formule (I) et du bromure d'ammonium ;
(iii) séparation du mélange réactionnel de l'acide aminocarboxylique de formule (I) et d'une solution aqueuse riche en bromure d'ammonium ;
(iv) mise en contact de la solution aqueuse riche en bromure d'ammonium obtenue avec de la soude pour former de l'ammoniac et une solution aqueuse riche en bromure de sodium ;
(v) purification de la solution aqueuse riche en bromure de sodium obtenue afin d'éliminer les impuretés organiques ;
(vi) mise en contact de la solution aqueuse riche en bromure de sodium purifiée obtenue avec du chlore pour former du brome et une solution aqueuse riche en chlorure de sodium ;
(vii) réaction du brome obtenu avec de l'hydrogène pour former du bromure d'hydrogène ; et
(viii) recyclage du bromure d'hydrogène obtenu vers l'étape (i).

Selon un mode de réalisation, on ajoute à la solution riche en bromure d'ammonium ou de sodium de l'étape (iv) des bromures provenant d'un autre procédé. Selon un autre mode de réalisation, on ajoute au brome obtenu à l'étape (vi) du brome provenant d'un autre procédé.

Selon un mode de réalisation, l'étape (v) de purification de la solution aqueuse de bromures permet de réduire le COT (carbone organique total) d'un facteur supérieur à 5. Selon un autre mode de réalisation, l'étape (vi) est opérée avec une solution de bromure ayant moins de 2%, et de préférence moins de 1% de COT et plus préférentiellement moins de 0.5% de COT.

Selon un premier mode de réalisation du procédé objet de l'invention, l'étape (v) est réalisée par acidification de la solution aqueuse riche en bromure de sodium issue de l'étape (iv) suivie d'une décantation de la phase huileuse formée. De préférence, la solution aqueuse riche en bromure de sodium issue de l'étape (iv) est acidifiée par ajout d'acide à un pH compris entre 3 et 5. Avantageusement, l'étape (v) comporte une étape (va) subséquente dans laquelle la phase huileuse obtenue à l'étape (v) est soumise à une opération d'extraction pour obtenir une solution aqueuse enrichie en bromure de sodium, laquelle est renvoyée vers l'étape (iv) ou (vi). Avantageusement, l'opération permettant d'extraire une solution aqueuse enrichie en bromure de sodium à l'étape (va) est une extraction liquide/liquide.

Selon un deuxième mode de réalisation du procédé objet de l'invention, l'étape (v) est réalisée par neutralisation de la solution aqueuse riche en bromure de sodium suivie d'une extraction liquide/liquide. De préférence, la solution aqueuse riche en bromure de sodium est neutralisée dans l'étape (v) jusqu'à un pH compris entre 3 et 10. Avantageusement, la neutralisation dans l'étape (v) est réalisée par ajout d'une solution aqueuse d'acide chlorhydrique, d'acide bromhydrique, d'acide sulfurique ou d'un de leurs mélanges.

Selon un troisième mode de réalisation du procédé objet de l'invention, l'étape (v) est réalisée par adsorption sur un matériau adsorbant. De préférence, le matériau adsorption est choisi parmi des matériaux minéraux tels que le charbon actif ; une silice, une alumine ou un mélange de ceux-ci ; ou des matériaux organiques comme les résines macroréticulées adsorbantes ou les résines échangeuses d'ions.

Selon un quatrième mode de réalisation du procédé objet de l'invention, l'étape (v) est réalisée par séparation membranaire pour former un perméat enrichi en bromure de sodium et appauvri en impuretés organiques et un concentrat appauvri en bromure de sodium et enrichi en impuretés organiques. L'étape (v) peut être réalisée notamment au moyen d'une ou plusieurs membranes de nanofiltration. Avantageusement, l'étape (v) comporte une étape (va') subséquente dans laquelle le concentrat appauvri en bromure de sodium et enrichi en impuretés organiques obtenu à l'étape (v) est soumis à une étape de diafiltration afin de récupérer une solution aqueuse enrichie en bromure de sodium et appauvrie en impuretés organiques, laquelle pourra être mélangée avec le perméat issu de l'étape (v). Par ailleurs, l'étape (v) peut comporter une étape (vb) subséquente dans laquelle le perméat enrichi en bromure de sodium et appauvri en impuretés organiques obtenu à l'étape (v) ainsi que, le cas échéant, la solution aqueuse enrichie en bromure de sodium et appauvrie en impuretés organiques issue de l'étape (va') sont soumis à une seconde étape de séparation membranaire.

### [Description des modes de réalisation]

### Définition des termes

On entend par le terme « **bromure** » désigner de manière générale un composé comprenant un atome de brome à l'état d'oxydation (-I) dépourvu de liaison carbone-hydrogène, tel qu'un sel de bromure, notamment un bromure d'alcalin tel que le bromure de sodium ou de potassium, le bromure d'ammonium ou encore le bromure d'hydrogène ou l'acide bromhydrique.

Comme évoqué plus haut, l'objet de la présente invention est d'améliorer l'économie du procédé de de production d'acides aminocarboxyliques tels que l'acide 12-aminododécanoïque, 11-aminoundécanoïque ou 10-décanoïque en réduisant, voire évitant l'apport de brome et les encrassements lors de la conversion du brome ainsi obtenu en bromure d'hydrogène.

Selon l'invention, le procédé de production d'acide aminocarboxylique de formule (I) suivante :

NH₂-CH₂-(CH₂)ₙ-COOH (I)

dans laquelle n est un entier de 7 à 12, de préférence de 8 à 10, comprend les étapes suivantes :
(i) réaction de l'acide carboxylique insaturé de formule (II) suivante :

   CH₂=CH-(CH₂)ₙ₋₁-COOH (II)

   avec du bromure d'hydrogène (HBr) pour former l'acide ω-bromoalcanoïque de formule (III) suivante:

   Br-CH₂-(CH₂)ₙ-COOH (III) ;
(ii) réaction de l'acide ω-bromoalcanoïque de formule (III) obtenu avec l'ammoniac en solution aqueuse pour former un mélange réactionnel comprenant l'acide aminocarboxylique de formule (I) et du bromure d'ammonium ;
(iii) séparation du mélange réactionnel de l'acide aminocarboxylique de formule (I) et d'une solution aqueuse riche en bromure d'ammonium ;
(iv) mise en contact de la solution aqueuse riche en bromure d'ammonium obtenue avec de la soude pour former de l'ammoniac et une solution aqueuse riche en bromure de sodium ;
(v) purification de la solution aqueuse riche en bromure de sodium obtenue afin d'éliminer les impuretés organiques ;
(vi) mise en contact de la solution aqueuse riche en bromure de sodium purifiée obtenue avec du chlore pour former du brome et une solution aqueuse riche en chlorure de sodium ;
(vii) réaction du brome obtenu avec de l'hydrogène pour former du bromure d'hydrogène ; et
(viii) recyclage du bromure d'hydrogène obtenu vers l'étape (i).

Comme expliqué ci-dessus, un certain nombre d'acides carboxyliques insaturés de formule (II) comme l'acide 11-dodécénoïque et l'acide 10-undécénoïque et l'acide 9-décénoïque mis en œuvre dans le procédé peuvent être obtenus de sources renouvelables telles que des huiles végétales.

L'acide 11-dodécénoïque peut être obtenu par réduction de l'acide 10-undécénoïque en undécénol, puis conversion en bromure d'undécényle, puis traitement au cyanure de sodium et hydrolyse du nitrile formé.

L'acide 10-undécénoïque peut être obtenu à partir de l'huile de ricin par les étapes suivantes : méthanolyse pour former le ricinoléate de méthyle, pyrolyse en 10-undécénoate de méthyle et heptanal, puis hydrolyse du 10-undécénoate de méthyle. L'acide 10-undécénoïque peut être également obtenu par pyrolyse de l'acide ricinoléique obtenu par hydrolyse de l'huile de ricin. Le ricinoléate de méthyle peut aussi être obtenu par trituration réactive de graines de ricin.

L'acide 9-décénoïque peut être obtenu à partir d'esters d'acides gras issus d'huiles naturelles par exemple par réaction avec un une alpha-oléfine en présence d'un catalyseur et hydrolyse de l'ester d'acide 9-décénoïque obtenu en acide 9-décénoïque, comme décrit dans WO 2018/080869.

### Etape (i)

La réaction de l'acide carboxylique insaturé de formule (II) avec du bromure d'hydrogène (HBr) selon l'étape (i) du procédé de l'invention pour former l'acide ω-bromoalcanoïque de formule (III) est connue de l'homme de l'art et décrite par exemple dans FR 951.932, US 2,772,302, EP 3 030 543 B1, CN 103804209 B ou WO 18/080869.

L'acide carboxylique insaturé de formule (II) est de préférence mis en œuvre sous forme liquide, notamment en fusion ou dissout dans un solvant ou un mélange de solvants.

Le HBr peut être sous forme gaz ou liquide, par exemple liquéfié sous sa pression de vapeur saturante ou en solution dans un solvant ou mélange de solvants.

Lorsqu'on utilise un solvant dans le procédé de l'invention, celui-ci est choisi de préférence parmi : benzène, fluorobenzène, chlorobenzène, toluène, α,α,α-trifluorotoluène, éthylbenzène, xylènes, cyclohexane, méthylcyclohexane, méthylcyclopentane, n-hexane, 2-méthylhexane, 3-méthylhexane, n-heptane, isooctane, tétrachloroéthylène, 1,1,1-trichloroéthane, dibromométhane, trichlorométhane, tétrachlorométhane, 1-bromopropane, carbonate de diméthyle, tétrahydrofurane (THF), 1,4-dioxane, 2-méthyltétrahydrofurane, tétrahydropyrane (THP), 1-propoxypropane, 1-ethoxybutane, 2-isopropoxypropane, acétonitrile, et leurs mélanges.

De manière avantageuse, le ou les solvants éventuels sont chauffés à une température comprise dans la gamme de -50°C à 30°C, de préférence de -40°C à 10°C, de préférence de -30°C à 0°C, de préférence de -30°C à -10°C.

On réalise la réaction généralement avec de l'HBr en excès molaire par rapport à l'acide carboxylique insaturé de formule (II).

La réaction est le plus souvent réalisée en présence d'un initiateur radicalaire. L'initiateur radicalaire est un générateur de radicaux qui peut être choisi notamment parmi l'oxygène ou un gaz contenant de l'oxygène tel l'air ou l'air enrichi en oxygène, un peroxyde comme par exemple le peroxyde de benzoyle, ou un rayonnement UV. En raison de sa facilité de mise en oeuvre industrielle, sa stabilité, et son coût moindre par rapport aux autres types d'initiateurs, l'oxygène ou un gaz contenant de l'oxygène est particulièrement préféré.

L'hydrobromuration peut être opérée en milieu liquide homogène, ou bien en phase liquide-gaz.

La réaction peut être réalisée dans un réacteur de type colonne à garnissage, un réacteur agité, un réacteur tubulaire.

Le mélange réactionnel sortant du réacteur peut subir une évaporation de l'HBr en excès ou peut être lavé à l'eau pour éliminer le HBr résiduel. Après l'évaporation du solvant éventuel et purification si nécessaire, on obtient l'acide ω-bromoalcanoïque de formule (III).

### Etapes (ii) et (iii)

Dans l'étape (ii) du procédé de l'invention, on fait réagir l'acide ω-bromoalcanoïque de formule (III) ainsi obtenu avec de l'ammoniac, étape appelée étape d'ammonolyse. L'étape (iii) du procédé de l'invention consiste à séparer du mélange réactionnel l'acide aminocarboxylique de formule (I) formé. La séparation peut être réalisée notamment par séparation solide liquide. Le produit est ensuite purifié, notamment par lavage avec de l'eau ou une solution aqueuse. On obtient ainsi des solutions aqueuses riches en bromure d'ammonium.

Ces étapes (ii) et (iii) sont connues de l'homme de l'art et sont décrites par exemple dans FR 928.265, FR 958.178, CN 1078585 C, CN 103804209B ou WO 18/080869.

On peut utiliser de l'ammoniac liquéfié ou de l'ammoniaque en solution aqueuse, alcoolique ou hydro-alcoolique. La réaction peut être conduite à température basse, par exemple à température ambiante ou 30°C, ou à température plus élevée, par exemple 80°C. Selon la température choisie, la durée de la réaction peut varier entre 5 et 120 heures environ, le rendement étant plus faible à température plus élevée. Avantageusement le milieu réactionnel est soumis à une montée en température régulière entre une température initiale de 15 à 25°C et une température finale de 26 à 40°C. La pression peut être la pression atmosphérique ou proche de celle-ci.

La réaction d'ammonolyse peut être réalisée dans un réacteur agité ou dans batterie de 2 à 25 réacteurs agités en série ou en parallèle.

L'acide aminocarboxylique de formule (I) formé peut être séparé du mélange réactionnel par exemple par les étapes suivantes. Le mélange réactionnel est dilué dans l'eau et chauffé à ébullition. L'ammoniac qui se dégage est recueilli dans l'eau pour former une solution d'ammoniaque, laquelle peut être réutilisée notamment pour fournir l'ammoniac utilisé à l'étape (ii). Le mélange est ensuite séparé de l'éventuelle couche d'huile formée par décantation à chaud avant d'être refroidi pour séparer l'acide 12-aminododécanoïque, l'acide 11-aminoundécanoïque ou l'acide 10-aminodécanoïque par cristallisation. Le solide est essoré, lavé à l'eau et éventuellement recristallisé dans l'eau bouillante.

Les eaux-mères recueillies pendant la séparation et la purification du produit visé contiennent du bromure d'ammonium et constituent la solution riche en bromure d'ammonium qui sera traitée par la suite dans le procédé de l'invention.

L'acide aminocarboxylique de formule (I) peut également être séparé du mélange réactionnel d'ammonolyse par séparation solide/liquide telle une filtration sur filtre ou un essorage. Les eaux mères recueillies peuvent subir une extraction liquide-liquide, une cristallisation et/ou une filtration pour former une solution aqueuse riche en bromure d'ammonium et appauvrie en acide aminocarboxylique de formule (I).

### Etape (iv)

Dans l'étape (iv), on fait réagir la solution aqueuse riche en bromure d'ammonium formée des eaux-mères obtenues à l'étape précédente avec de la soude pour transformer le bromure d'ammonium en bromure de sodium, avec dégagement d'ammoniac sous forme gazeuse.

Cette réaction peut être réalisée par exemple dans un réacteur agité ou une colonne garnie.

La quantité de soude ajoutée sera avantageusement approximativement stœchiométrique par rapport à la quantité d'ions ammonium. Un résultat très satisfaisant est obtenu avec une soude ajoutée en ratio molaire de 1 : 0.8 à 1 : 1,25 par rapport à la teneur de la solution en ions ammonium. Avantageusement, la quantité de soude ajoutée à cette étape est une quantité stœchiométrique ou en léger excès, par exemple un ratio molaire de 1 : 1.0 à 1 : 1.1 par rapport à la teneur de la solution en ions ammonium.

La soude peut être ajoutée sous forme solide ou sous forme de solution, notamment aqueuse. Avantageusement, on utilise une solution aqueuse de soude concentrée afin de réduire les consommations d'eau. La mise en œuvre de soude sous forme de solution présentant une teneur de 2 à 20 mol/l, et notamment de 6 à 11 mol/l est particulièrement utile.

Le mélange réactionnel peut être amené à température plus élevée pour favoriser l'évaporation de l'ammoniac. De préférence, on chauffe le mélange réactionnel à une température de 50 à 150°C, avantageusement de 80 à 130°C et encore préféré de 95 à 110°C de façon à évaporer l'ammoniac et une partie de l'eau. L'évaporation de l'ammoniac permet d'éviter la formation de produits secondaires potentiellement gênants dans les étapes subséquentes du procédé.

L'ammoniac dégagé est avantageusement réutilisé dans l'étape (ii), soit tel quel, soit après dissolution dans de l'eau.

La solution aqueuse riche en bromure de sodium récupérée présente généralement un pH de 9 à 13, et de préférence 9,5 à 11,5.

### Etape (v)

Dans cette étape, la solution aqueuse riche en bromure de sodium obtenue à l'étape précédente est soumise à une ou plusieurs étapes de purification afin d'éliminer les impuretés organiques. Ces impuretés forment un mélange complexe dont la teneur peut être caractérisée par le carbone organique total (COT).

Le fait d'éliminer les impuretés organiques de la solution aqueuse riche en bromure de sodium permet de pouvoir opérer l'étape (vi) sans encrassement par des suies qui sont très pénalisantes pour l'exploitation d'un tel procédé.

Les impuretés organiques présentes dans la solution de bromure de sodium peuvent être éliminées notamment par acidification, extraction liquide-liquide en milieu acide ou neutre, par adsorption ou par séparation membranaire, ou une combinaison quelconque entre ces méthodes. Ces quatre modes de réalisation A - D de l'étape (v) sont décrits plus en détail ci-dessous.

### Etape (v) Variante A : Acidification puis décantation

Dans cette variante, la solution de bromure de sodium issue de l'étape (iv) est acidifiée par l'ajout d'un acide à un pH inférieur à 5. Il se forme alors une phase huileuse moins dense, laquelle peut être séparée aisément, par exemple par décantation.

La solution riche en bromure de sodium issue de l'étape (iv) peut être acidifiée par exemple par ajout d'une solution aqueuse d'un acide minéral fort comme l'acide chlorhydrique, l'acide bromhydrique ou l'acide sulfurique, seul ou en mélange. De préférence, la solution riche en bromure de sodium acidifiée présente un pH compris de 3 à 5 et de préférence 3 à 4,5.

On observe la formation d'une phase huileuse riche en impuretés organiques qui est moins dense que la solution aqueuse riche en bromure de sodium. La phase huileuse peut être séparée par un moyen connu en soi, par exemple par décantation. Cette décantation peut être opérée par exemple dans un décanteur statique ou un décanteur centrifuge. La solution aqueuse riche en bromure de sodium purifiée, appauvrie en composés organiques, peut ensuite être envoyée à l'étape (vi), éventuellement après une ou plusieurs étapes de purification additionnelles selon l'une des variantes A-D.

La phase huileuse formée est éliminée directement ou soumise au préalable à une ou plusieurs étapes d'extraction des bromures de façon à pouvoir récupérer une phase aqueuse contenant des bromures et la renvoyer vers l'étape (v) ou vers l'étape (vi) tout en éliminant une phase huileuse contenant les impuretés organiques.

Selon un mode de réalisation, la phase huileuse peut être soumise à une extraction liquide-liquide, par exemple avec une solution aqueuse saline concentrée, afin d'extraire davantage de bromures.

Particulièrement préférée est l'extraction au moyen d'une solution de chlorure de sodium, de préférence une telle solution présentant une concentration de 1 mol/l à 6 mol/l. On récupère une phase aqueuse contenant des bromures qui est renvoyée vers l'étape (v) ou vers l'étape (vi) et une phase huileuse qui est éliminée.

En variante, l'extraction des bromures de la phase huileuse peut être réalisée par mélange avec de l'eau et un acide carboxylique liquide immiscible à l'eau et décantation. A titre de tels acides carboxyliques immiscibles, on peut citer l'acide heptanoïque, l'acide octanoïque, l'acide 2-éthyl hexanoïque, l'acide nonanoïque, un acide gras comportant 10 à 12 atomes de carbone ou un mélange de tels acides. On peut également utiliser des résidus de distillation (légers et lourds) de l'acide heptanoïque ou l'acide octanoïque ou l'acide 2-éthyl hexanoïque ou l'acide nonanoïque, de préférence de tels résidus contenant au moins 30% et de préférence au moins 50% d'acide heptanoïque, d'acide octanoïque, d'acide 2-éthyl hexanoïque ou d'acide nonanoïque.

Le ratio massique phase huileuse / eau / acide carboxylique peut varier de 1:0.3:0.3 à 1:3:2. On opère cette extraction généralement entre la température ambiante et 130°C et de préférence entre 50 et 100°C.

L'extraction peut être réalisée par exemple dans un réacteur agité avec une étape de mise en contact par mélange vigoureux. On peut aussi utiliser un mélangeur statique. On obtient une phase huileuse appauvrie en bromures et une solution aqueuse enrichie en bromures, lesquelles peuvent être séparées par un moyen classique de séparation liquide-liquide, par exemple par décantation, réalisé dans le réacteur ou dans un décanteur.

En alternative, on peut réaliser l'extraction dans une colonne continue d'extraction liquide-liquide avec :
- injection de l'eau dans la partie supérieure de la colonne ;
- injection de l'acide carboxylique et de la phase huileuse dans la partie inférieure de la colonne ;
- soutirage de la phase aqueuse dans la partie inférieure de la colonne ; et
- soutirage de la phase huileuse dans la partie supérieure de la colonne.

La solution aqueuse riche en bromure de sodium purifiée obtenue peut être envoyée vers l'étape (v) ou vers l'étape (vi), éventuellement après une ou plusieurs étapes de purification additionnelles selon l'une des variantes A-D.

Selon un autre mode de réalisation, l'extraction des bromures de la phase huileuse peut être réalisé par une étape de diafiltration par dilution à l'eau ou avec une solution aqueuse de soude et séparation membranaire. En sortie de la diafiltration, on obtient un flux enrichi en impuretés organiques et appauvri en bromure de sodium et une phase aqueuse enrichie en bromures et appauvrie en impuretés organiques. Le flux enrichi en impuretés organiques et appauvri en bromures peut être éliminé. La phase aqueuse enrichie en bromures et appauvrie en impuretés organiques peut être envoyée vers l'étape (v) ou vers l'étape (vi).

Les membranes utilisées et la façon d'opérer sont décrites dans la variante D ci-après. Avantageusement, le pH du mélange peut être ajusté préalablement à l'envoi sur la membrane à un pH supérieur à 7, de préférence à 8 à 11 et de préférence 9 à 10.

### Etape (v) Variante B : Extraction liquide-liquide

Dans cette variante, la solution aqueuse riche en bromure de sodium issue de l'étape (iv) est neutralisée, puis soumise directement, c'est-à-dire sans décantation préalable, à une extraction liquide-liquide.

La solution aqueuse riche en bromure de sodium issue de l'étape (iv) peut être neutralisée par exemple par ajout d'une solution aqueuse d'un acide minéral fort comme l'acide chlorhydrique, l'acide bromhydrique ou l'acide sulfurique, seul ou en mélange. De préférence, la solution riche en bromure de sodium neutralisée présente un pH compris de 3 à 10 et de préférence 4 à 10.

La solution aqueuse riche en bromure de sodium peut être ensuite directement soumise à une extraction liquide-liquide telle que décrite ci-dessus. En particulier, la solution riche en bromure de sodium peut être mélangée avec un acide carboxylique immiscible à l'eau tels que ceux mentionnés ci-dessus. La phase huileuse formée peut être séparée de manière classique par exemple par décantation.

L'extraction liquide/liquide peut aussi être réalisée dans un réacteur agité, ou dans une colonne continue d'extraction liquide-liquide avec :
- injection de la solution riche en bromure de sodium dans la partie supérieure de la colonne ;
- injection de l'acide carboxylique dans la partie inférieure de la colonne ;
- soutirage de la solution aqueuse riche en bromure de sodium purifiée dans la partie inférieure de la colonne ; et
- soutirage de la phase huileuse dans la partie supérieure de la colonne.

La phase huileuse formée est éliminée directement ou est soumise au préalable à une ou plusieurs étapes additionnelles d'extraction des bromures.

La solution aqueuse riche en bromure de sodium purifiée obtenue peut subir une étape d'entrainement à la vapeur d'eau de façon à en éliminer des impuretés organiques volatiles. Cette étape d'entrainement à la vapeur peut être réalisée dans un réacteur agité ou dans une colonne.

La solution aqueuse riche en bromure de sodium purifiée obtenue peut être envoyée vers l'étape (vi), éventuellement après une ou plusieurs étapes de purification additionnelles selon l'une des variantes A-D.

### Etape (v) Variante C : Adsorption

Dans cette variante, la solution aqueuse riche en bromure de sodium issue de l'étape (iv) est purifiée par mise en contact avec un adsorbant.

Un adsorbant adapté peut être notamment le charbon actif, une résine macroréticulée adsorbante, une résine échange d'ion ou un mélange de résines échangeuses d'ions, un adsorbant de type silice, de type alumine ou du type silice-alumine.

La mise en contact peut être réalisée dans une ou plusieurs colonnes ou bien dans un réacteur agité et suivie d'une séparation solide / liquide. Avantageusement, la mise en contact est réalisée dans plusieurs colonnes en série dans lesquelles le solide est fixe et le liquide traverse le lit de solide. Lorsque l'adsorbant est saturé, la solution aqueuse riche en bromures peut être envoyée sur un autre réacteur ou une ou plusieurs autres colonnes comportant un adsorbant frais. L'adsorbant saturé peut être déchargé et éliminé ou le cas échéant régénéré.

L'adsorbant peut être régénéré de manière conventionnelle, par exemple au moyen d'un lavage à l'eau, avec un solvant miscible avec l'eau puis à nouveau à l'eau. Le solvant chargé des impuretés organiques extraites de l'adsorbant et d'eau peut être régénéré par distillation et réutilisé. Comme exemple de solvants on peut citer le méthanol, l'éthanol, le diméthylsulfoxyde, l'acide acétique ou l'acide propanoïque.

Au préalable de l'étape d'adsorption, le pH de la solution aqueuse de bromure de sodium peut être ajusté, notamment à un pH de 4 et de 11 et de préférence entre 8 et 10.5, par exemple au moyen d'un ajout d'acide comme expliqué ci-dessus, de façon à être dans la gamme optimale de l'efficacité de l'adsorbant.

La solution aqueuse riche en bromure de sodium purifiée obtenue peut être envoyée vers l'étape (vi), éventuellement après une ou plusieurs étapes de purification additionnelles selon l'une des variantes A-D.

### Etape (v) Variante D : Séparation membranaire

Dans cette variante, la solution aqueuse riche en bromure de sodium issue de l'étape (iv) est purifiée par séparation membranaire.

La séparation membranaire peut être réalisée par exemple par nanofiltration, par pervaporation, ou par osmose inverse.

Les membranes peuvent être réalisées en céramique, en verre ou métal, en composite, ou encore en polymère réticulé ou non, ou encore être mixtes (inorganiques ou organiques). Les membranes en polyamide comme les membranes de nanofiltration MPS-34 commercialisées par la société Koch Membrane Systems sont particulièrement préférées.

La membrane peut être opérée en mode continu, en semi-continu ou en discontinu. Le flux peut être frontal ou tangentiel par rapport au filtre.

La solution aqueuse riche en bromure de sodium est envoyée sur une membrane, ce qui permet de séparer un premier flux appauvri en impuretés organiques d'un second flux enrichi en impuretés organiques.

Afin d'augmenter encore le taux de récupération des bromures, le flux enrichi en impuretés organiques peut être ensuite soumis à une étape de diafiltration par dilution à l'eau et séparation membranaire. En sortie de la diafiltration, on obtient un flux enrichi en impuretés organiques et appauvri en bromure de sodium et un flux enrichi de bromures et appauvri en impuretés organiques. Le flux enrichi en impuretés organiques et appauvri en bromure peut être éliminé. Le flux enrichi en bromure et appauvri en impuretés organiques peut être mélangé avec le premier flux de bromures appauvri en impuretés organiques.

Préalablement au passage sur membrane, le pH de la solution aqueuse riche en bromure de sodium peut être ajusté à un pH supérieur à 7, de préférence à 8 à 11 et de préférence 9 à 10, par exemple au moyen d'un ajout de base, par exemple de soude, ou d'un acide comme expliqué ci-dessus, de façon à optimiser l'efficacité des membranes en termes de séparation ainsi que leur durée de vie.

En variante, le flux enrichi en impuretés organiques peut être soumis, à la place de l'étape de diafiltration, à une étape d'extraction des bromures au moyen d'une ou plusieurs des étapes décrites dans les variantes A - C ci-dessus.

La solution aqueuse purifiée obtenue après la séparation membranaire peut ensuite être envoyée vers l'étape (vi), éventuellement après une ou plusieurs étapes de purification additionnelles selon l'une des variantes A-D.

### Etape (vi)

Cette étape vise à transformer en brome les bromures dans la solution aqueuse riche en bromure de sodium purifiée obtenue à l'étape précédente. Cette étape est réalisée par réaction de la solution aqueuse avec du chlore, de sorte à obtenir du brome et une solution aqueuse riche en chlorure de sodium.

L'étape peut être réalisée par exemple dans une colonne comportant un garnissage, avec :
- injection en tête de colonne de la solution aqueuse de bromure de sodium purifiée issue de l'étape (v),
- injection de chlore dans le quart inférieur de la colonne ;
- injection de la vapeur d'eau en pied de colonne de façon à chauffer le pied de colonne à une température de 70 à 100°C, avantageusement de 90 et 100°C ;
- soutirage en pied de colonne d'un flux composé principalement de chlorure de sodium et d'eau.
- récupération en tête de colonne du brome.

Avantageusement, la solution aqueuse de bromure de sodium contient moins de 2% et de préférence moins de 1% et plus préférentiellement moins de 0.5% de carbone organique total (COT). En effet, il a été constaté qu'une telle concentration permet de limiter les pertes en bromure en pied de colonne.

Le débit de chlore est avantageusement ajusté de façon à assurer une quantité stœchiométrique et de préférence un excès molaire de 0 à 30%, encore préféré de 5 à 20% par rapport aux bromures injectés.

### Etape (vii)

Cette étape vise à faire réagir le brome obtenu avec de l'hydrogène pour former du bromure d'hydrogène. Cette étape est connue par l'homme du métier et décrite par exemple dans « *Bromine and its compounds »,* édité par Z.E. Jolles (London 1966) page 82, le brevet US 2,070,263 ou dans *«* Bromine » de Ullmann's encyclopedia of Industrial Chemistry, édité par Wiley (2015).

Plus spécifiquement, cette étape peut être conduite par condensation du brome récupéré en tête de colonne à l'étape précédente, et injection sous forme vaporisée dans un réacteur tubulaire à co-courant avec de l'hydrogène porté à une température élevée, par exemple 500 à 1000°C.

On récupère ainsi du bromure d'hydrogène issu partiellement voire complètement du recyclage de bromures produites dans le procédé.

On a découvert que l'on a d'autant moins d'encrassements dans le réacteur de synthèse de bromure d'hydrogène sous forme de suies lorsque la solution aqueuse de bromure qui entre dans l'étape (v) contient moins de COT.

### Etape (vii)

Cette étape vise à recycler le bromure d'hydrogène obtenu à l'étape précédente vers l'étape (i).

Le bromure d'hydrogène formé est refroidi et peut être réutilisé pour l'étape d'hydrobromuration décrite à l'exemple 1.

Dans certains modes de réalisation du procédé, la quantité de bromure d'hydrogène récupérée à l'étape (vii) est inférieure à la quantité de bromure d'hydrogène injectée à l'étape (i). De façon à ce que le bromure d'hydrogène produit soit en quantité au moins égale au bromure d'hydrogène dont on a besoin à l'étape (i), un appoint en élément brome peut être effectué à différents points du procédé, notamment sous forme de brome à l'étape (vi), ou sous forme de bromure à l'étape (iv) ou (v) ou A, B, C ou D.

Selon un mode de réalisation particulier, l'acide aminocarboxylique de formule (I) obtenu à l'étape (iii) peut subir des étapes additionnelles de purification telles une dissolution suivie d'une extraction liquide/liquide, d'une adsorption, une recristallisation, un séchage.

L'acide aminocarboxylique éventuellement ainsi purifié peut être polymérisé, par exemple par polycondensation, en polyamide correspondant. En alternative, il peut également être mis en œuvre avec d'autres monomères comme par exemple des polyéthers pour la fabrication de copolymères correspondants

L'invention sera expliquée plus en détail dans les exemples qui suivent.

### [Exemples]

### Mesure du carbone organique total (COT)

Le dosage du carbone organique total est réalisé par différence entre le carbone total mesuré par la méthode de combustion catalytique à 680°C avec détection infrarouge et le carbone inorganique mesuré par acidification à l'acide chlorhydrique 2N et détection infrarouge, au moyen d'un COT mètre Shimadzu.

### Mesure de la perte en brome en pied de colonne de synthèse du brome

On quantifie les pertes en brome à l'étape de synthèse du brome en rapportant le débit de bromures dans le flux de saumure en pied de colonne au débit de bromures injectés dans la colonne. Les débits de bromures sont calculés par le produit du débit massique du flux par la concentration massique en bromures mesurée par argentimétrie, selon le protocole suivant : la solution à titrer est diluée dans de l'eau distillée acidifiée par une goutte d'acide nitrique puis titrée par une solution aqueuse de nitrate d'argent (0.1 mol/l) à l'aide d'un dispositif de titration Mettler Titrator équipé d'une électrode DMi141-SC.

### Encrassement du réacteur de synthèse du bromure d'hydrogène

La formation de suies dans le réacteur de synthèse de bromure d'hydrogène est détectée par une observation visuelle qualitative après un jour de fonctionnement du réacteur tubulaire.

### Exemple 1 : Préparation d'acide 11-bromoundécanoïque par hydrobromuration d'acide 10-undécénoïque

Un mélange de solvant benzène / toluène avec un ratio volumique de 50/50 est envoyé en continu en tête d'une colonne d'absorption refroidie à -20°C où on injecte en pied du bromure d'hydrogène gazeux.

Le flux de bromure d'hydrogène et de solvant récupéré en pied de la colonne est mélangé à un flux d'oxygène et mis en contact avec un flux d'acide 10-undécénoïque à 50°C dans un dispositif de mélange en T connecté à un tuyau en PFA.

Les ratios massiques des débits respectifs de solvant, bromure d'hydrogène, oxygène et acide 10-undécénoïque sont 6,3/0,49/0,0028/1. Le volume du tuyau correspond à un temps de séjour de 0,4 minutes.

Le mélange réactionnel sortant de ce tuyau est évaporé de façon à séparer les solvants benzène et toluène et le bromure d'hydrogène en excès et à isoler l'acide 11-bromoundécanoïque, qui présente une pureté voisine de 94%.

### Exemple 2 : Préparation d'acide 11-aminoundécanoïque par ammonolyse d'acide 11-bromoundécanoïque

Dans un réacteur double enveloppe muni d'une agitation mécanique, on place de l'ammoniaque à 32% à 0°C. On ajoute, à pression atmosphérique, par un goutte-à-goutte rapide, 220 g d'acide 11-bromoundécanoïque fondu à 90°C. Le ratio massique entre l'ammoniaque à 32% et l'acide 11-bromoundécanoïque est de 6/1. La consigne de température du milieu est ajustée à 22°C puis toutes les 12h30 on impose au milieu réactionnel une montée en température de 2°C jusqu'au sixième palier à 32°C qui dure également 12h30.

### Exemple 3 : Séparation de l'acide 11-aminoundécanoïque et traitement des filtrats

Le milieu réactionnel obtenu à l'exemple 2 est filtré et le gâteau est lavé avec un peu d'eau. Le gâteau est mis en suspension dans de l'eau telle que la masse d'eau utilisée soit 3.8 fois supérieure à la quantité d'acide 11-bromoundécanoïque utilisé. Le mélange est recristallisé par chauffage à 120°C puis refroidissement à 25°C. Après filtration, le gâteau est lavé avec un peu d'eau. Le solide récupéré est séché pour obtenir une poudre d'acide 11-aminoundécanoïque.

L'ensemble des filtrats est rassemblé puis porté à 95°C et évaporé sous pression réduite. On arrête l'évaporation lorsqu'on atteint un extrait sec d'environ 50% puis on refroidit à 25°C. Après séparation solide-liquide, le gâteau d'acide 11-aminoundécanoïque est lavé à l'eau.

Les filtrats de cette étape de cristallisation, comprenant du bromure d'ammonium et des impuretés organiques en solution dans l'eau, sont évaporés jusqu'à une concentration en bromures d'environ 350 g/l.

On ajoute alors de la soude en solution aqueuse à 50% massique en quantité appropriée pour atteindre un pH de 11 et on chauffe à 95°C de façon à ce que l'ammoniac se dégage et soit piégé dans de l'eau.

La solution aqueuse récupérée contient du bromure de sodium et des impuretés organiques. La solution contient 3% de carbone organique total.

### Exemple 4 : Traitement des bromures de sodium par le chlore

Dans une colonne comportant un garnissage, on injecte en continu en tête de la colonne la solution aqueuse de bromure de sodium issue de l'exemple 3 ci-dessus, du chlore dans le quart inférieur de la colonne et de la vapeur d'eau en pied de colonne de façon à chauffer le pied de colonne à 100°C. Le débit de chlore est ajusté de façon à avoir un excès molaire de 10% par rapport aux bromures injectés.

On récupère en pied de colonne un flux de saumure composé principalement de chlorure de sodium et d'eau. Le brome récupéré en tête de colonne est condensé, puis vaporisé et injecté à co-courant avec de l'hydrogène dans un réacteur tubulaire porté à 900°C.

Le bromure d'hydrogène formé est refroidi et peut être réutilisé pour l'étape d'hydrobromuration décrite à l'exemple 1.

Les pertes en bromes dans la colonne de synthèse du brome et l'encrassement du réacteur de synthèse du bromure d'hydrogène sont reportés au tableau 1.

### Exemple 5 : Traitement des bromures de sodium par le chlore avec élimination des impuretés organiques préalable (Procédé A)

La solution aqueuse de bromure de sodium obtenue à l'exemple 3 ci-dessus, refroidie à 25°C, est acidifiée avec de l'acide chlorhydrique à 33% de façon à atteindre un pH de 4. La phase huileuse qui décante est séparée. La phase aqueuse, qui est analysée à 0.3% de carbone organique total, est ensuite traitée comme dans l'exemple 4.

Les pertes en bromes dans la colonne de synthèse du brome et l'encrassement du réacteur de synthèse du bromure d'hydrogène sont reportés au tableau 1.

**[Tableau 1]**

| Exemple | COT de phase aqueuse de NaBr [%] | Perte en brome [%] en pied de la colonne de synthèse du Br2 | Encrassement du réacteur de synthèse de l'HBr |
|---|---|---|---|
| 4 | 3 | 3 | Beaucoup de suies |
| 5 | 0.3 | 0.2 | Pas de suies visibles |
| 6 | 0.1 | 0.1 | Pas de suies visibles |
| 7 | 0.03 | <0.1 | Pas de suies visibles |
| 8 | 0.2 | 0.1 | Pas de suies visibles |

### Exemple 6 : Traitement des bromures de sodium par le chlore avec élimination des impuretés organiques préalable (Procédé C)

La solution aqueuse de bromure de sodium de l'exemple 3 et une résine macroréticulée Amberlite XAD 4 dans le rapport massique 5:1 sont agités pendant 15 minutes puis on opère une séparation solide/liquide. La phase aqueuse de bromure de sodium, dont le carbone organique total est de 0.1% est ensuite traitée comme décrit dans l'exemple 4. Les pertes en bromes dans la colonne de synthèse du brome et l'encrassement du réacteur de synthèse du bromure d'hydrogène sont reportés au tableau 1.

### Exemple 7 : Traitement des bromures de sodium par le chlore avec élimination des impuretés organiques préalable (Procédé D)

La solution aqueuse de bromure de sodium de l'exemple 3 est envoyée à 50°C et 30 bar sur une membrane de nanofiltration MPS-34 (Koch Membrane Systems). Le facteur de concentration volumique, défini comme le rapport du débit volumique d'alimentation sur le débit volumique de concentrat, est d'environ 10. Le débit de filtration moyen mesuré est de 6 kg/h/m2. Le carbone organique total mesuré pour le perméat est de 0,03%.

La quantité de brome perdue dans le concentrat (ratio de la quantité de bromures dans le concentrat sur la quantité de bromures dans la solution aqueuse de bromure de sodium envoyée sur la nanofiltration) est de 5,4%.

Une étape de diafiltration sur la même membrane est réalisée sur le concentrat après dilution avec 0.5 volume d'eau pour 1 volume de concentrat. Cette deuxième étape de séparation membranaire permet de baisser les pertes en bromures à 1,4% des bromures de la solution aqueuse de bromure de sodium envoyée sur l'étape de nanofiltration.

Les perméats rassemblés sont ensuite traités comme décrit dans l'exemple 4. Les pertes en bromes dans la colonne de synthèse du brome et l'encrassement du réacteur de synthèse du bromure d'hydrogène sont reportés au tableau 1.

### Exemple 8 : Traitement des bromures de sodium par le chlore avec élimination des impuretés organiques préalable (Procédé D)

La solution aqueuse de bromure de sodium de l'exemple 3 est envoyée à 50°C et 30 bar sur une membrane de nanofiltration DK de Suez. Le débit de filtration moyen mesuré est de 24 kg/h/m2. Le carbone organique total mesuré pour le perméat est de 0,2%. La quantité de brome perdue dans le concentrat sans étape de diafiltration est de 0.3%.

Le perméat est ensuite traité comme décrit dans l'exemple 4. Les pertes en bromes dans la colonne de synthèse du brome et l'encrassement du réacteur de synthèse du bromure d'hydrogène sont reportés au tableau 1.

L'ensemble des résultats met en évidence que le procédé selon l'invention permet de récupérer une quantité importante de bromures dans les effluents du procédé de fabrication d'acide 11-aminoundécanoïque et d'acide 10-aminodécanoïque, réduisant ainsi le besoin d'ajout de brome, ce qui améliore notablement l'économie du procédé tout en réduisant par ailleurs la quantité d'effluents.

### [Liste des documents cités]

US 2009/0292073 A1
US 2005/0004326 A1
US 8,013,251 B2
US 2017/0242372 A1

## Revendications

1. Un procédé de production d'acide aminocarboxylique de formule (I) suivante :
NH₂CH₂-(CH₂)ₙ-COOH (I)
dans laquelle n est un entier de 7 à 12, de préférence de 8 à 10, comprenant les étapes suivantes :
(i) réaction de l'acide carboxylique insaturé de formule (II) suivante :
CH₂=CH-(CH₂)ₙ₋₁-COOH (II)
avec du bromure d'hydrogène (HBr) pour former l'acide ω-bromoalcanoïque de formule (III) suivante :
Br-CH₂-(CH₂)ₙ-COOH (III) ;
(ii) réaction de l'acide ω-bromoalcanoïque de formule (III) obtenu avec l'ammoniac en solution aqueuse pour former un mélange réactionnel comprenant l'acide aminocarboxylique de formule (I) et du bromure d'ammonium ;
(iii) séparation du mélange réactionnel de l'acide aminocarboxylique de formule (I) et d'une solution aqueuse riche en bromure d'ammonium ;
(iv) mise en contact de la solution aqueuse riche en bromure d'ammonium obtenue avec de la soude pour former de l'ammoniac et une solution aqueuse riche en bromure de sodium ;
(v) purification de la solution aqueuse riche en bromure de sodium obtenue afin d'éliminer les impuretés organiques ;
(vi) mise en contact de la solution aqueuse riche en bromure de sodium purifiée obtenue avec du chlore pour former du brome et une solution aqueuse riche en chlorure de sodium ;
(vii) réaction du brome obtenu avec de l'hydrogène pour former du bromure d'hydrogène ; et
(viii) recyclage du bromure d'hydrogène obtenu vers l'étape (i).

2. Procédé selon la revendication 1, dans lequel on ajoute à la solution riche en bromure d'ammonium ou de sodium de l'étape (iv) des bromures provenant d'un autre procédé.

3. Procédé selon la revendication 1 ou 2, dans lequel on ajoute au brome obtenu à l'étape (vi) du brome provenant d'un autre procédé.

4. Procédé selon l'une des revendications 1 à 3 dans laquelle l'étape (v) de purification de la solution aqueuse de bromures permet de réduire le COT d'un facteur supérieur à 5.

5. Procédé selon l'une des revendications 1 à 4 dans laquelle l'étape (vi) est opérée avec une solution de bromure ayant moins de 2%, et de préférence moins de 1% et avantageusement moins de 0.5% de COT.

6. Procédé selon l'une des revendications 1 à 5, dans lequel l'étape (v) est réalisée par acidification de la solution aqueuse riche en bromure de sodium issue de l'étape (iv) suivie d'une décantation de la phase huileuse formée.

7. Procédé selon la revendication 6, dans lequel la solution aqueuse riche en bromure de sodium issue de l'étape (iv) est acidifiée par ajout d'acide à un pH compris entre 3 et 5.

8. Procédé selon l'une des revendications 1 à 7, dans lequel l'étape (v) comporte une étape (va) subséquente dans laquelle la phase huileuse formée à l'étape (v) est soumise à une opération d'extraction pour obtenir une solution aqueuse enrichie en bromure de sodium, laquelle est renvoyée vers l'étape (iv) ou (vi), notamment par extraction liquide/liquide.

9. Procédé selon l'une des revendications 1 à 5, dans lequel l'étape (v) est réalisée par neutralisation de la solution aqueuse riche en bromure de sodium suivie d'une extraction liquide/liquide.

10. Procédé selon la revendication 9, dans lequel la solution aqueuse riche en bromure de sodium est neutralisée dans l'étape (v) jusqu'à un pH compris entre 3 et 10, notamment par ajout d'une solution aqueuse d'acide chlorhydrique, d'acide bromhydrique, d'acide sulfurique ou d'un de leurs mélanges.

11. Procédé selon l'une des revendications 1 à 5, dans lequel l'étape (v) est réalisée par adsorption sur un matériau adsorbant, notamment choisi parmi des matériaux minéraux tels que le charbon actif ; une silice, une alumine ou un mélange de ceux-ci ; ou des matériaux organiques comme les résines macroréticulées adsorbantes ou les résines échangeuses d'ions.

12. Procédé selon l'une des revendications 1 à 5, dans lequel l'étape (v) est réalisée par séparation membranaire pour former un perméat enrichi en bromure de sodium et appauvri en impuretés organiques et un concentrat appauvri en bromure de sodium et enrichi en impuretés organiques.

13. Procédé selon la revendication 12, dans lequel l'étape (v) est réalisée au moyen d'une ou plusieurs membranes de nanofiltration.

14. Procédé selon la revendication 12 ou 13, dans lequel l'étape (v) comporte une étape (va') subséquente dans laquelle le concentrat appauvri en bromure de sodium et enrichi en impuretés organiques obtenu à l'étape (v) est soumis à une étape de diafiltration afin de récupérer une solution aqueuse enrichie en bromure de sodium et appauvrie en impuretés organiques, laquelle pourra être mélangée avec le perméat issu de l'étape (v).

15. Procédé selon l'une des revendications 12 à 14, dans lequel l'étape (v) comporte une étape (vb) subséquente dans laquelle le perméat enrichi en bromure de sodium et appauvri en impuretés organiques obtenu à l'étape (v) ainsi que, le cas échéant, la solution aqueuse enrichie en bromure de sodium et appauvrie en impuretés organiques issue de l'étape (va') sont soumis à une seconde étape de séparation membranaire.

## Patentansprüche

1. Verfahren zur Herstellung einer Aminocarbonsäure der folgenden Formel (I):
NH₂-CH₂-(CH₂)ₙ-COOH (I),
wobei n eine ganze Zahl von 7 bis 12, vorzugsweise von 8 bis 10, ist, das die folgenden Schritte umfasst:
(i) die Umsetzung einer ungesättigten Carbonsäure der folgenden Formel (II):
CH₂=CH-(CH₂)ₙ₋₁-COOH (II)
mit Bromwasserstoff (HBr), um die ω-Bromalkansäure der folgenden Formel (III) zu bilden:
Br-CH₂-(CH₂)ₙ-COOH (III);
(ii) die Umsetzung der erhaltenen ω-Bromalkansäure der Formel (III) mit Ammoniak in wässriger Lösung, um eine Reaktionsmischung zu bilden, welche die Aminocarbonsäure der Formel (I) und Ammoniumbromid umfasst;
(iii) die Trennung der Reaktionsmischung der Aminocarbonsäure der Formel (I) und einer an Ammoniumbromid reichen wässrigen Lösung;
(iv) das In-Kontakt-Bringen der erhaltenen an Ammoniumbromid reichen wässrigen Lösung mit Natriumhydroxid, um Ammoniak und eine an Natriumbromid reiche wässrige Lösung zu bilden;
(v) das Reinigen der erhaltenen an Natriumbromid reichen wässrigen Lösung, um die organischen Verunreinigungen zu entfernen;
(vi) das In-Kontakt-Bringen der erhaltenen gereinigten, an Ammoniumbromid reichen wässrigen Lösung mit Chlor, um Brom und eine an Natriumchlorid reiche wässrige Lösung zu bilden;
(vii) die Umsetzung des erhaltenen Broms mit Wasserstoff, um Bromwasserstoff zu bilden; und
(viii) das Rückführen des erhaltenen Bromwasserstoffs in Schritt (i).

2. Verfahren nach Anspruch 1, wobei von einem anderen Verfahren stammende Bromide zu der an Ammonium- oder Natriumbromid reichen Lösung von Schritt (iv) gegeben werden.

3. Verfahren nach Anspruch 1 oder 2, wobei von einem anderen Verfahren stammendes Brom zu dem in Schritt (vi) erhaltenen Brom gegeben wird.

4. Verfahren nach einem der Ansprüche 1 bis 3, wobei Schritt (v) der Reinigung der wässrigen Bromidlösung eine Verminderung des TOC um einen Faktor von mehr als 5 ermöglicht.

5. Verfahren nach einem der Ansprüche 1 bis 4, wobei Schritt (vi) mit einer Bromidlösung erfolgt, die weniger als 2 %, vorzugsweise weniger als 1% und vorteilhafterweise weniger als 0,5 % TOC aufweist.

6. Verfahren nach einem der Ansprüche 1 bis 5, wobei Schritt(v) durch Ansäuern der an Natriumbromid reichen wässrigen Lösung aus Schritt (iv), gefolgt von einem Dekantieren der gebildeten öligen Phase, erfolgt.

7. Verfahren nach Anspruch 6, wobei die an Natriumbromid reiche wässrige Lösung aus Schritt (iv) durch die Zugabe einer Säure auf einen pH-Wert zwischen 3 und 5 angesäuert wird.

8. Verfahren nach einem der Ansprüche 1 bis 7, wobei Schritt (v) einen nachfolgenden Schritt (va) aufweist, in dem die in Schritt (v) gebildete ölige Phase einem Extraktionsvorgang unterzogen wird, um insbesondere durch eine Flüssig-Flüssig-Extraktion eine mit Natriumbromid angereicherte wässrige Lösung zu erhalten, die in Schritt (iv) oder (vi) zurückgeführt wird.

9. Verfahren nach einem der Ansprüche 1 bis 5, wobei Schritt(v) durch eine Neutralisation der an Natriumbromid reichen wässrigen Lösung, gefolgt von einer Flüssig-Flüssig-Extraktion, erfolgt.

10. Verfahren nach Anspruch 9, wobei die an Natriumbromid reiche wässrige Lösung in Schritt (v) bis zu einem pH-Wert zwischen 3 und 10 insbesondere durch die Zugabe einer wässrigen Salzsäure-, Bromwasserstoffsäure-, Schwefelsäurelösung oder einer Mischung davon neutralisiert wird.

11. Verfahren nach einem der Ansprüche 1 bis 5, wobei Schritt (v) durch eine Adsorption an einem adsorbierenden Material erfolgt, das insbesondere aus mineralischen Materialien, wie Aktivkohle; einem Siliciumdioxid, einem Aluminiumoxid oder einer Mischung davon oder organischen Materialien, wie adsorbierenden makrovernetzten Harzen oder Ionenaustausch-Harzen, ausgewählt ist.

12. Verfahren nach einem der Ansprüche 1 bis 5, wobei Schritt (v) durch eine Membrantrennung erfolgt, um ein mit Natriumbromid angereichertes und an organischen Verunreinigungen verarmtes Permeat und ein an Natriumbromid verarmtes und mit organischen Verunreinigungen angereichertes Konzentrat zu bilden.

13. Verfahren nach Anspruch 12, wobei Schritt (v) mittels einer oder mehrerer Nanofiltrationsmembranen erfolgt.

14. Verfahren nach Anspruch 12 oder 13, wobei Schritt (v) einen nachfolgenden Schritt (va') aufweist, in dem das an Natriumbromid verarmte und mit organischen Verunreinigungen angereicherte Konzentrat, das in Schritt (v) erhalten wird, einem Diafiltrationsschritt unterzogen wird, um eine mit Natriumbromid angereicherte und an organischen Verunreinigungen verarmte wässrige Lösung zu gewinnen, die mit dem Permeat aus Schritt (v) mischbar ist.

15. Verfahren nach einem der Ansprüche 12 bis 14, wobei Schritt (v) einen nachfolgenden Schritt (vb) aufweist, in dem das mit Natriumbromid angereicherte und an organischen Verunreinigungen verarmte Permeat, das in Schritt (v) erhalten wird, sowie gegebenenfalls die mit Natriumbromid angereicherte und an organischen Verunreinigungen verarmte wässrige Lösung aus Schritt (va') einem zweiten Membrantrennschritt unterzogen werden.

## Claims

1. Process for producing an aminocarboxylic acid of formula (I) below:
NH₂-CH₂-(CH₂)ₙ-COOH (I)
in which n is an integer from 7 to 12, preferably from 8 to 10, comprising the following steps:
(i) reacting the unsaturated carboxylic acid of formula (II) below:
CH₂=CH-(CH₂)ₙ₋₁-COOH (II)
with hydrogen bromide (HBr) to form the ω-bromoalkanoic acid of formula (III) below:
Br-CH₂-(CH₂)ₙ-COOH (III);
(ii) reacting the ω-bromoalkanoic acid of formula (III) obtained with ammonia in aqueous solution to form a reaction mixture comprising the aminocarboxylic acid of formula (I) and ammonium bromide;
(iii) separating the reaction mixture of the aminocarboxylic acid of formula (I) and an aqueous solution rich in ammonium bromide;
(iv) bringing the aqueous solution rich in ammonium bromide obtained into contact with sodium hydroxide to form ammonia and an aqueous solution rich in sodium bromide;
(v) purifying the aqueous solution rich in sodium bromide obtained in order to eliminate the organic impurities;
(vi) bringing the purified aqueous solution rich in sodium bromide obtained into contact with chlorine to form bromine and an aqueous solution rich in sodium chloride;
(vii) reacting the bromine obtained with hydrogen to form hydrogen bromide; and
(viii) recycling the hydrogen bromide obtained to step (i).

2. Process according to Claim 1, in which bromides originating from another process are added to the solution rich in ammonium bromide or sodium bromide of step (iv).

3. Process according to Claim 1 or 2, in which bromine originating from another process is added to the bromine obtained in step (vi).

4. Process according to one of Claims 1 to 3, in which step (v) of purifying the aqueous solution of bromides makes it possible to reduce the TOC by a factor of more than 5.

5. Process according to one of Claims 1 to 4, in which step (vi) is performed with a bromide solution having less than 2%, and preferably less than 1% and advantageously less than 0.5% of TOC.

6. Process according to one of Claims 1 to 5, in which step (v) is carried out by acidification of the aqueous solution rich in sodium bromide resulting from step (iv) followed by a decantation of the oily phase formed.

7. Process according to Claim 6, in which the aqueous solution rich in sodium bromide resulting from step (iv) is acidified by addition of acid to a pH of between 3 and 5.

8. Process according to one of Claims 1 to 7, in which step (v) comprises a subsequent step (va) in which the oily phase formed in step (v) is subjected to an extraction operation to obtain an aqueous solution enriched in sodium bromide, which is sent back to step (iv) or (vi), in particular by liquid/liquid extraction.

9. Process according to one of Claims 1 to 5, in which step (v) is carried out by neutralization of the aqueous solution rich in sodium bromide followed by a liquid/liquid extraction.

10. Process according to Claim 9, in which the aqueous solution rich in sodium bromide is neutralized in step (v) to a pH between 3 and 10, in particular by addition of an aqueous solution of hydrochloric acid, of hydrobromic acid, of sulfuric acid or of a mixture thereof.

11. Process according to one of Claims 1 to 5, in which step (v) is carried out by adsorption on an adsorbent material, in particular chosen from mineral materials such as activated carbon; a silica or an alumina or a mixture thereof; organic materials such as adsorbent macrocrosslinked resins or ionexchange resins.

12. Process according to one of Claims 1 to 5, in which step (v) is carried out by membrane separation to form a permeate enriched in sodium bromide and depleted in organic impurities and a concentrate depleted in sodium bromide and enriched in organic impurities.

13. Process according to Claim 12, in which step (v) is carried out by means of one or more nanofiltration membranes.

14. Process according to Claim 12 or 13, in which step (v) comprises a subsequent step (va') in which the concentrate depleted in sodium bromide and enriched in organic impurities which is obtained in step (v) is subjected to a step of diafiltration in order to recover an aqueous solution enriched in sodium bromide and depleted in organic impurities, which solution will be able to be mixed with the permeate resulting from step (v).

15. Process according to one of Claims 12 to 14, in which step (v) comprises a subsequent step (vb) in which the permeate enriched in sodium bromide and depleted in organic impurities which is obtained in step (v) and also, where appropriate, the aqueous solution enriched in sodium bromide and depleted in organic impurities resulting from step (va') are subjected to a second step of membrane separation.
